# EUROPEAN PATENT SPECIFICATION

(11) **EP 3 600 343 B1**
(45) Date of publication and mention of the grant of the patent: **23.11.2022**
(21) Application number: 18770433.3
(22) Date of filing: 26.03.2018
(51) Int. Cl.: A61K 31/7088, A61K 48/00

(54) **NOVEL RECOMBINANT ADENO-ASSOCIATED VIRAL VECTORS RESTRICTING OFF-TARGET TRANSDUCTION IN LIVER AND USES THEREOF**
NEUARTIGE REKOMBINANTE ADENO-ASSOZIIERTE VIRALE VEKTOREN ZUR EINSCHRÄNKUNG DER OFF-TARGET-TRANSDUKTION IN DER LEBER UND VERWENDUNGEN DAVON
NOUVEAUX VECTEURS VIRAUX ADÉNO-ASSOCIÉS RECOMBINÉS LIMITANT LA TRANSDUCTION HORS CIBLE DANS LE FOIE ET LEURS UTILISATIONS

(30) Priority: 24.03.2017 US 201762476290 P
(43) Date of publication of application: 05.02.2020
(73) Proprietor: Ohio State Innovation Foundation, Columbus, OH 43201 (US)
(72) Inventor: CAO, Lei, Columbus Ohio 43220 (US); HUANG, Wei, Worthington Ohio 43085 (US)
(74) Representative: Creek, Isobel Clare
(86) International application number: PCT/US2018/024305
(87) International publication number: WO 2018/176027

(56) References cited:
- WO-A2-02/22175
- US-A1- 2003 215 423
- US-A1- 2009 111 766
- US-A1- 2011 288 160
- WEI HUANG ET AL: "Targeting Visceral Fat by Intraperitoneal Delivery of Novel AAV Serotype Vector Restricting Off-Target Transduction in Liver", MOLECULAR THERAPY - METHODS & CLINICAL DEVELOP, vol. 6, 1 September 2017 (2017-09-01), pages 68-78, XP055753046, GB ISSN: 2329-0501, DOI: 10.1016/j.omtm.2017.06.002
- S M O'NEILL ET AL: "Targeting adipose tissue via systemic gene therapy", GENE THERAPY, vol. 21, no. 7, 15 May 2014 (2014-05-15), pages 653-661, XP055753460, GB ISSN: 0969-7128, DOI: 10.1038/gt.2014.38
- XIANGLAN LIU ET AL: "Adipose tissue insulin receptor knockdown via a new primate-derived hybrid recombinant AAV serotype", MOLECULAR THERAPY - METHODS & CLINICAL DEVELOP, vol. 1, no. 8, 1 January 2014 (2014-01-01) , page 8, XP055753139, GB ISSN: 2329-0501, DOI: 10.1038/mtm.2013.8
- RUN XIAO ET AL: "Adipocytes: A Novel Target for IL-15/IL-15R[alpha] Cancer Gene Therapy", MOLECULAR THERAPY, vol. 27, no. 5, 1 May 2019 (2019-05-01), pages 922-932, XP055753477, US ISSN: 1525-0016, DOI: 10.1016/j.ymthe.2019.02.011

## Description

1. This Application claims the benefit of U.S. Provisional Application No. 62/476,290, filed on March 24, 2017. This invention was made with government support under Grant No. CA163640 and CA166590 awarded by the National Cancer Institute of the National Institutes of Health. The government has certain rights in the invention.

### I. BACKGROUND

2. Adipose tissue is a multifunctional organ that modulates whole body metabolic homeostasis. Germline genetic manipulation based on transgenic techniques is often used for experimental studies of adipose tissue. But some research and therapeutic applications require adipose manipulation with gene delivery at certain age. In this regard, viral vectors become an attractive alternative delivery vehicle, in particular, recombinant adeno-associated virus (rAAV) vectors because they can transduce both dividing and postmitotic tissues with low immunogenicity and long-lasting transgene expression without stimulating cell-mediated immune response. rAAVs have gained more attention for their successful applications in clinical trials. In addition, rAAVs are widely applied in basic research for gene delivery to multiple tissues including liver, heart, skeleton muscle, brain and eyes. Yet applications of rAAVs for targeting adipose tissue have been limited due to low transduction efficiency of naturally occurring AAV serotypes.

3. Modifications of the capsid or the expression cassette are employed to improve efficiency of gene delivery and transgene tropism with either local or systematic delivery. For example, selecting more efficient naturally occurring serotypes plus the use of adipose tissue specific promoter or micro-RNA targeting sequence enable specific transduction of adipose tissue, but require high dose to achieve therapeutic effects. Moreover, expression of recombinant therapies in off target tissues can have deleterious effects. What are needed are new high transduction efficiency viral vectors that can avoid off target deleterious effects.

### II. SUMMARY

4. Disclosed are methods and compositions related to novel recombinant adeno-associated viral vectors. The invention is defined by the appended claims.

5. In one aspect disclosed herein is an hybrid serotype Rec2 engineered adeno-associated virus (AAV) vector comprising two expression cassettes; wherein the first cassette comprises a regulatory element and a transgene operatively linked to a promoter; and wherein the second cassette comprises a first tissue-specific promoter operatively linked to a RNA silencing element that targets the regulatory element in the first expression cassette. In one aspect, expression of the second cassette restricts off-target transduction of the transgene in the first tissue.

6. Also disclosed are engineered AAV of any preceding aspect, wherein the regulatory element of the first cassette is a woodchuck posttranscriptional regulatory element (WPRE) sequence.

7. Also disclosed are engineered AAV of any preceding aspect, wherein the transgene of the first cassette is a leptin gene or a IL-15 gene.

8. Also disclosed are engineered AAV of any preceding aspect, wherein the tissue specific promoter of the second cassette is a liver specific promoter.

9. In one aspect, disclosed herein are engineered AAV of any preceding aspect for use in methods of treating diabetes, obesity, metabolic syndrome, cancer, or lipodystrophy in a subject, comprising administering to the subject the adeno-associated viral vector of any preceding aspect.

10. In one aspect, disclosed are engineered AAV of any preceding aspect for use in of targeting a nucleic acid based treatment to visceral adipose tissue in a subject comprising administering to the subject an engineered adeno-associated virus (AAV) vector of any preceding aspect intraperitoneally.

### III. BRIEF DESCRIPTION OF THE DRAWINGS

11. The accompanying drawings, which are incorporated in and constitute a part of this specification, illustrate several embodiments and together with the description illustrate the disclosed compositions and methods.

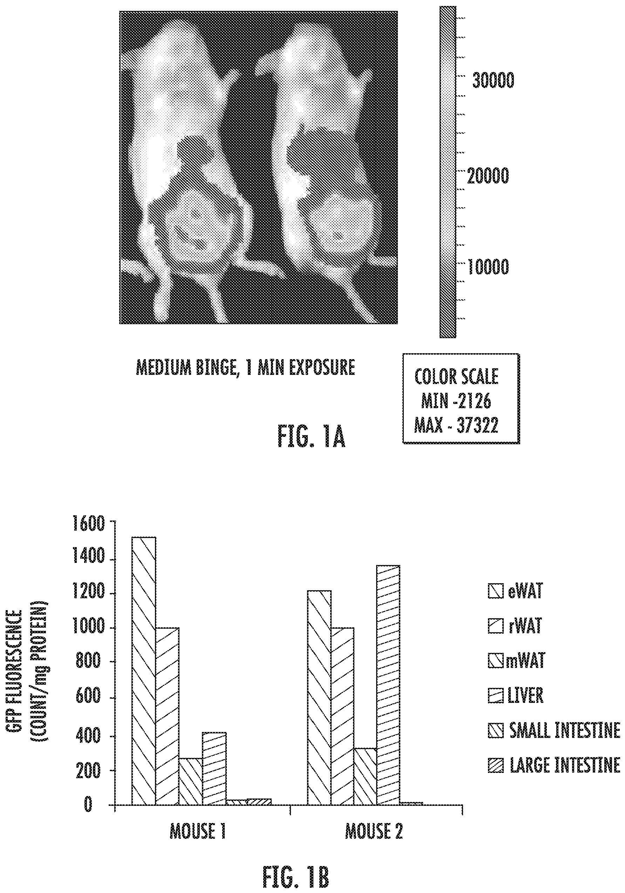

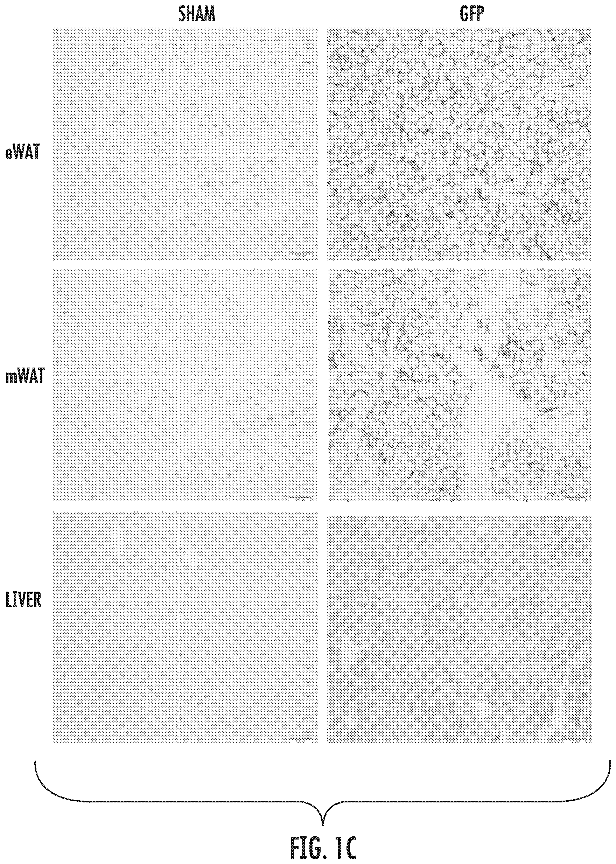

12. Figures 1A, 1B, and 1C show that IP administration of Rec2 vector transduces multiple visceral fat depots. (A) Representative *in vivo* bioluminescence imaging of luciferase 2 weeks after IP Rec2-luciferase vector injection (2×10¹⁰ vg per mouse). (B) GFP content in tissue lysates of mice receiving Rec2-GFP (1×10¹⁰ vg per mouse) 2 weeks after IP injection. (C) GFP immunohistochemistry in mice receiving Rec2-GFP (1×10¹⁰ vg per mouse). Scale bar: 100 µm.

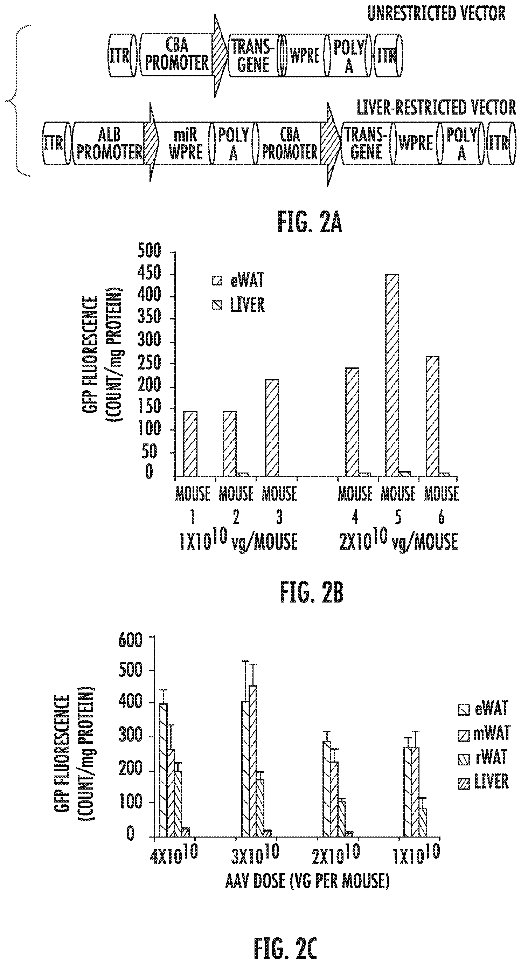

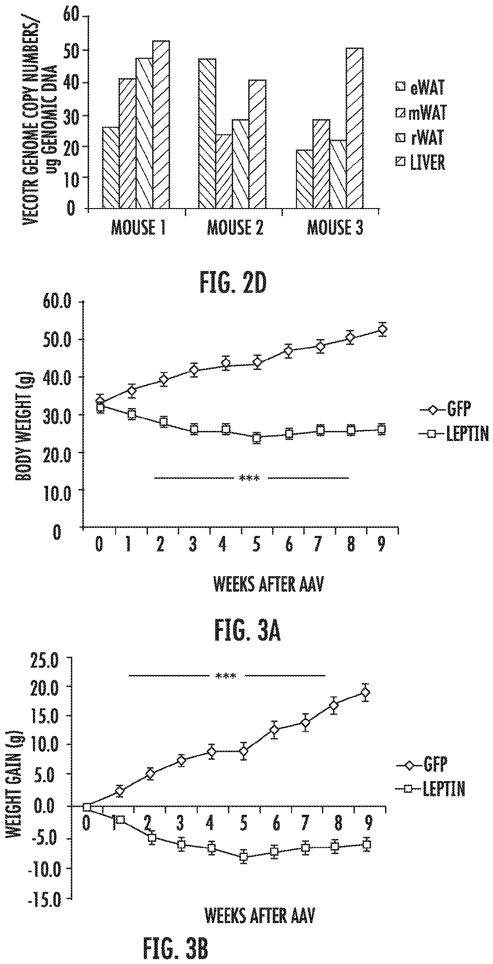

13. Figures 2A, 2B, 2C, and 2D show that IP administration of dual-cassette AS/Rec2 vector selectively transduces visceral fat while restricting off-target transgene expression in liver. (A) Schematic of the AAV vectors. The liver-restricted vector contains two expression cassettes, one to express transgene under non-selective CBA promoter, the other to express a microRNA targeting WPRE driven by liver-specific albumin (Alb) promoter thereby preventing transgene expression in liver. (B) GFP content in tissue lysates of mice receiving IP injection of AS/Rec2-GFP at indicated doses of one experiment 2 weeks after IP injection. (C) GFP content in tissue lysates in another dose-response experiment 2 weeks after IP injection. Data are means±SEM. n=3-4 per group. GFP undetectable in small intestine, large intestine, spleen, kidney, testis, brown fat, or subcutaneous fat. (D) Viral vector genomic copy numbers in selected tissues 2 weeks after IP administration of AS/Rec2-GFP (4×10¹⁰ vg per mouse).

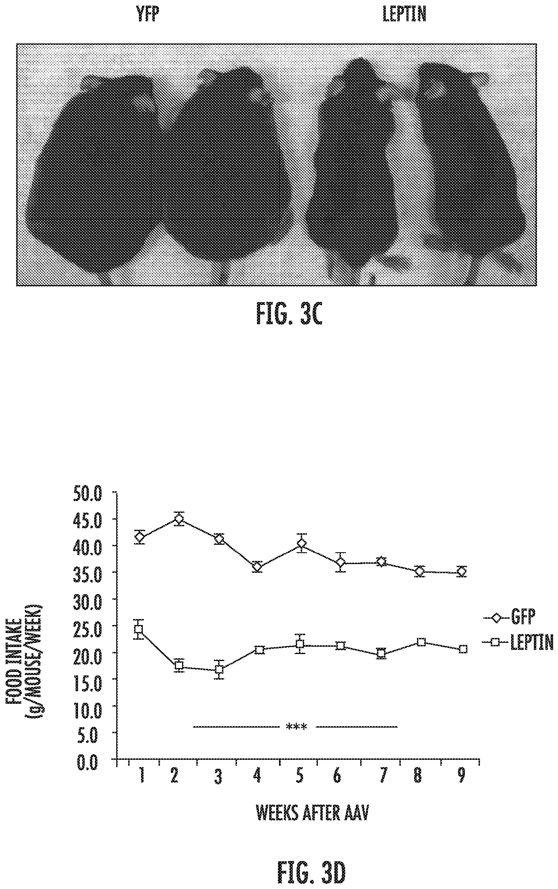

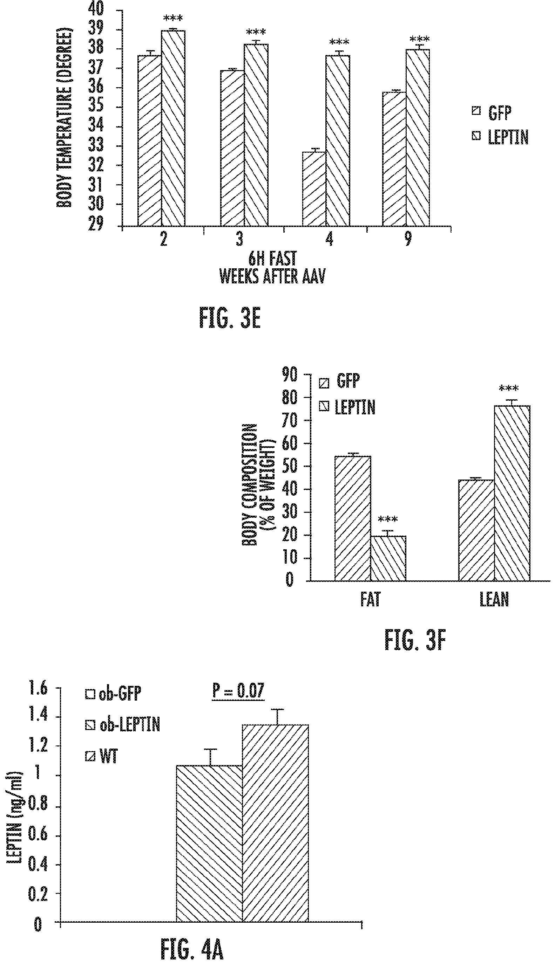

14. Figures 3A, 3B, 3C, 3D, 3E, and 3F show that IP administration of AS/Rec2-leptin vector rescues the leptin-deficient phenotype of *ob*/*ob* mice. (A) Body weight. (B) Weigh gain. (C) Representative picture 9 weeks after AAV injection. (D) Cumulative food intake. (E) Rectal temperature. At 4-week time point, body temperature was measured after 6 h fast. (F) EchoMRI analysis of body composition 4 weeks after AAV injection. Data are means±SEM. n=6 per group. ^{∗∗∗} P<0.001.

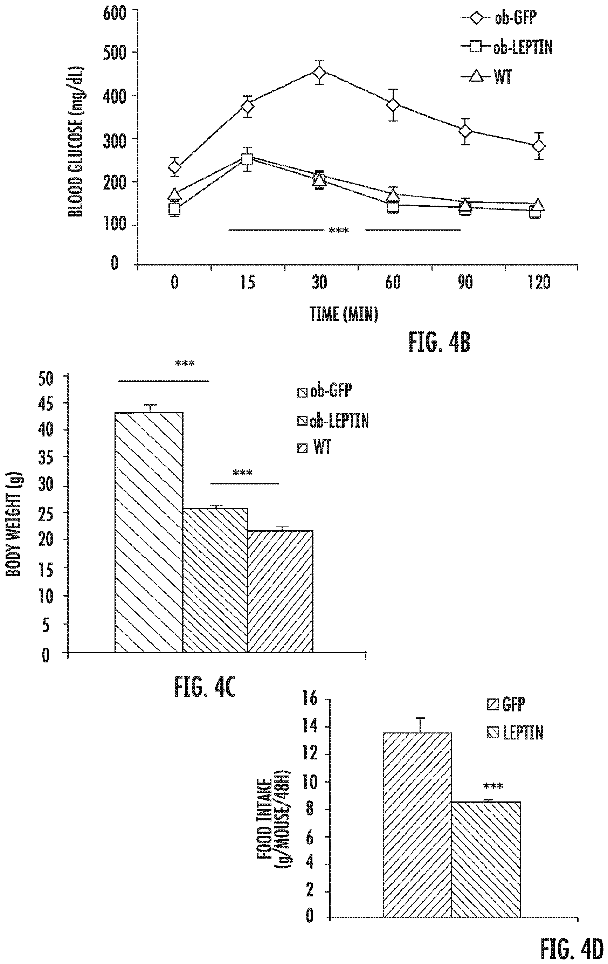

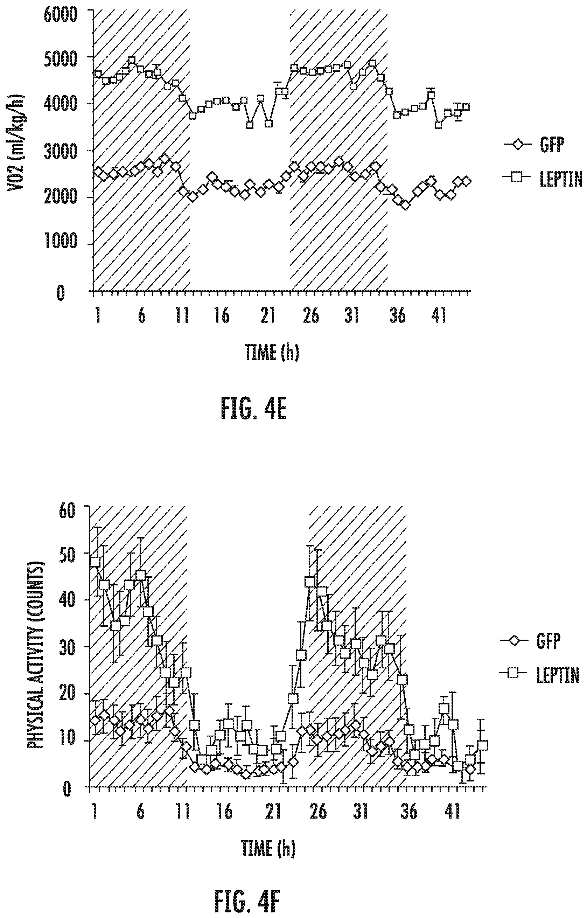

15. Figures 4A, 4B, 4C, 4D, 4E, and 4F show that AS/Rec2-leptin treatment corrects impaired glucose tolerance and disturbance of energy balance in *ob*/*ob* mice. (A) Serum leptin level 4 weeks after AAV injection. Leptin undetectable in AS/Rec2-GFP-treated *ob*/*ob* mice. (B) Glucose tolerance test 4 weeks after AAV injection. ^{∗∗∗} P<0.001 AS/Rec2-leptin-treated *ob*/*ob* mice and WT mice compared to AS/Rec2-GFP-treated *ob*/*ob* mice. At 0 min and 60 min, glucose levels in AS/Rec2-leptin-treated *ob*/*ob* mice were significantly lower than WT mice *(P<0.05).* (C) Body weight at glucose tolerance test. (D) Cumulative food. (E) Oxygen consumption. P<0.01 (F) Physical activity measured by CLAMS 5 weeks after AAV injection, P<0.05. Shaded area: dark phase. Data are means±SEM. n=6 AS/Rec2-GFP, n=6 AS/Rec2-leptin, n=6-8 age-matched WT.

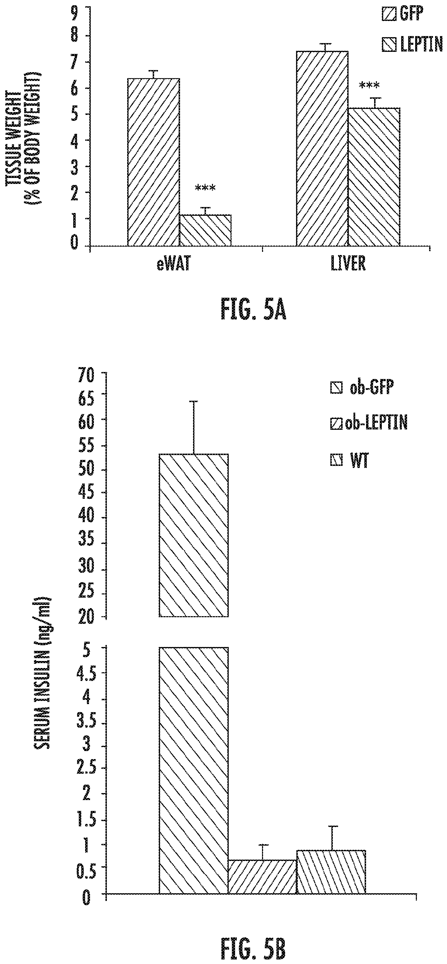

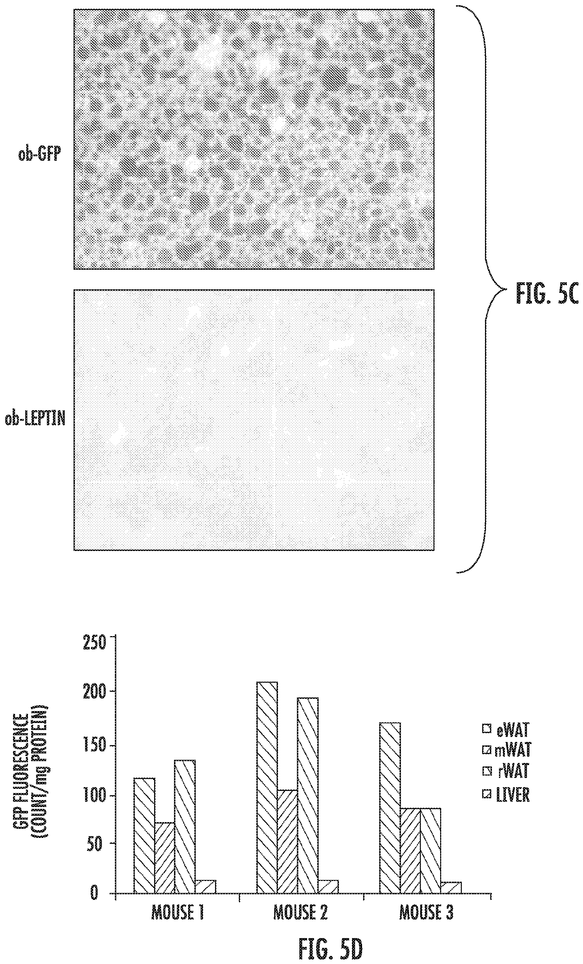

16. Figure 5A, 5B, 5C, and 5D show that AS/Rec2-leptin treatment reverses obesity, hyperinsulinemia, and liver steatosis in *ob*/*ob* mice. (A) Relative tissue weight at sacrifice 9 weeks after AAV injection. (B) Serum insulin level at sacrifice. (C) Oil Red O staining of livers. Scale bar: 50 µm. (D) GFP content in tissue lysates of individual *ob*/*ob* mouse receiving AS/Rec2-GFP.

### IV. DETAILED DESCRIPTION

17. Before the present compounds, compositions, articles, devices, and/or methods are disclosed and described, it is to be understood that they are not limited to specific synthetic methods or specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

### A. Definitions

18. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

19. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10"as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

20. In this specification and in the claims which follow, reference will be made to a number of terms which shall be defined to have the following meanings:

21. "Optional" or "optionally" means that the subsequently described event or circumstance may or may not occur, and that the description includes instances where said event or circumstance occurs and instances where it does not.

22. Throughout this application, various publications are referenced. The disclosures of these publications describe the state of the art.

### B. Compositions

23. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular adeno-associated viral vector is disclosed and discussed and a number of modifications that can be made to a number of molecules including the adeno-associated viral vector are discussed, specifically contemplated is each and every combination and permutation of the adeno-associated viral vector and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the subgroup of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

24. Obesity and body fat distribution are important risk factors for type II diabetes, lipodystrophy, and other metabolic syndromes. Visceral adipose tissue (VAT) plays distinctive roles in metabolic homeostasis and disturbance. The type of obesity characterized by increased VAT is strongly associated with adverse metabolic outcomes whereas accumulation of subcutaneous fat is thought to have neutral or even beneficial effects on metabolism. This relates to intrinsic functional differences of adipocytes in different depots, including insulin sensitivity, glucose uptake, rate of lipolysis, and adipokine and cytokine secretion. Thus, a gene delivery tool targeting visceral fat has great potentials for applications in basic research and gene therapy.

25. There are a number of compositions and methods which can be used to deliver nucleic acids to cells, either *in vitro* or *in vivo.* These methods and compositions can largely be broken down into two classes: viral based delivery systems and non-viral based delivery systems. For example, the nucleic acids can be delivered through a number of direct delivery systems such as, electroporation, lipofection, calcium phosphate precipitation, plasmids, viral vectors, viral nucleic acids, phage nucleic acids, phages, cosmids, or via transfer of genetic material in cells or carriers such as cationic liposomes.

26. As used herein, plasmid or viral vectors are agents that transport a nucleic acid of interest, such as leptin or IL-15 into the cell without degradation and include a promoter yielding expression of the gene in the cells into which it is delivered. Viral vectors are, for example, Adenovirus, Adeno-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Retroviruses include Murine Maloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, they are not as useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature.

27. Viral vectors can have higher transaction (ability to introduce genes) abilities than chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promotor cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans.

28. Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An especially preferred embodiment of this type of vector is the P4.1 C vector produced by Avigen, San Francisco, CA, which can contain the herpes simplex virus thymidine kinase gene, HSV-tk, and/or a marker gene, such as the gene encoding the green fluorescent protein, GFP.

29. In another type of AAV virus, the AAV contains a pair of inverted terminal repeats (ITRs) which flank at least one cassette containing a promoter which directs cell-specific expression operably linked to a heterologous gene. Heterologous in this context refers to any nucleotide sequence or gene which is not native to the AAV or B19 parvovirus.

30. Typically the AAV and B19 coding regions have been deleted, resulting in a safe, noncytotoxic vector. The AAV ITRs, or modifications thereof, confer infectivity and site-specific integration, but not cytotoxicity, and the promoter directs cell-specific expression. United States Patent No. 6,261,834 is herein incorproated by reference for material related to the AAV vector.

31. Herein is disclosed a novel engineered hybrid serotype Rec2 vector which leads to high transduction of adipose tissue superior to naturally occurring serotypes (AAV1 and AAV8) and other engineered serotypes (Rec1, Rec3, Rec4) when the vectors are injected directly to the fat pads at a dose 1 to 2 orders lower than previously reported studies. Interestingly, the administration route substantially influences the tropism and efficacy of Rec2 vector. Intravenous administration of Rec2 vector primarily transduces liver at the doses ranging from 2×10⁹ to 2×10¹⁰ vg per mouse. In contrast, oral administration of Rec2 vector leads to preferential transduction of brown fat with absence of transduction in the gastrointestinal track at the doses lower than 2×10¹⁰ vg per mouse. To improve gene delivery to VAT, it was firstly determined whether Rec2 vector, through IP administration, a simple and less invasive procedure, could efficiently transduce VAT. Accordingly, in one aspect, disclosed herein are methods of targeting a nucleic acid based treatment to visceral adipose tissue in a subject comprising administering to the subject any of the engineered adeno-associated virus (AAV) vectors disclosed herein (for example, the disclosed Rec2 vector). For example, disclosed herein are methods of targeting a nucleic acid based treatment to visceral adipose tissue in a subject comprising administering to the subject an engineered adeno-associated virus (AAV) vector comprising two expression cassettes; wherein the first cassette comprises a regulatory element and a leptin transgene or IL-15 transgene operatively linked to a promoter; and wherein the AAV vector is administered intraperitoneally.

32. To prevent transgene expression in an off-target tissue, such as the liver, a new AAV expression plasmid was generated that contained two expression cassettes: one comprising the transgene (for example, a nucleic acid encoding leptin or IL-15), and the other cassette used the liver-specific promoter (for example, the albumin promoter) to drive a specific RNA silencing element (such as, for example, microRNA) targeting a regulatory element (such as, for example, WPRE) sequence which only exists in the transgene expression cassette.

33. The inserted genes in viral and retroviral usually contain promoters, and/or enhancers to help control the expression of the desired gene product (for example, a chicken β-actin (CBA) promoter). A promoter is generally a sequence or sequences of DNA that function when in a relatively fixed location in regard to the transcription start site. A promoter contains core elements required for basic interaction of RNA polymerase and transcription factors, and may contain upstream elements and response elements.

34. Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. a β-actin promoter, such as, for example, the chicken β-actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a HindIII E restriction fragment (Greenway, P.J. et al., Gene 18: 355-360 (1982)). Of course, promoters from the host cell or related species also are useful herein. Thus, in one aspect, disclosed herein are engineered adeno-associated virus (AAV) vector comprising two expression cassettes; wherein the first cassette comprises a regulatory element and a transgene operatively linked to a promoter (such as, for example, the chicken β-actin promoter); and wherein the second cassette comprises a liver-specific albumin promoter and microRNA that targets the regulatory element in the first expression cassette.

35. Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al.,Mol. Cell Bio. 4: 1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, -fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

36. The promotor and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

37. In certain embodiments the promoter and/or enhancer region can act as a constitutive promoter and/or enhancer to maximize expression of the region of the transcription unit to be transcribed. In certain constructs the promoter and/or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTR.

38. It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin. In another aspect, a liver-specific promoter such as the albumin promoter, thyroxine binding globin promoter, phosphoenolpyruvatecarboxykinase (PEPCK) promoter, tyrosine aminotransgerase (TAT) promoter or human alpha 1-antitrypsin (hAAT) promoter can be used to drive expression only in the liver. By using, in the second cassette of the engineered AAV vectors disclosed herein, tissue specific promoters operatively linked to the RNA silencing element, expression of an RNA silencing element (such as, for example microRNA) which targets the regulatory element in the first cassette can be used to limit off-target transduction of the transgene (for example leptin and/or IL-15). For example, disclosed herein are engineered adeno-associated virus (AAV) vectors comprising two expression cassettes; wherein the first cassette comprises a regulatory element and a transgene (such as, for example, leptin and/or IL-15) operatively linked to a promoter (such as, for example CBA); and wherein the second cassette comprises a tissue specific promoter, for example, a liver specific promoter (such as, for example, albumin or hAAT) and a RNA silencing element (such as, for example, microRNA) that targets the regulatory element in the first expression cassette. It is understood and herein contemplated that the tissue-specific promoter such as the liver specific promoter in the second cassette is operatively linked to the RNA silencing element such that transcription of the RNA silencing element only occurs when in the appropriate tissue for the tissue specific promoter. Thus, as noted above, the disclosed engineered AAV can restrict off-target transduction of a transgene in the liver. Thus, in one aspect, disclosed herein are methods of expressing a transgene in the VAT while restricting off-target transduction of the transgene in the liver comprising administering to subject an engineered adeno-associated virus (AAV) vectors comprising two expression cassettes; wherein the first cassette comprises a regulatory element and a transgene (such as, for example, leptin and/or IL-15) operatively linked to a promoter (such as, for example CBA); and wherein the second cassette comprises a liver specific promoter (such as, for example albumin or hAAT) and a RNA silencing element (such as, for example, microRNA) that targets the regulatory element in the first expression cassette; and wherein the RNA silencing element is operatively linked to the liver specific promoter.

39. Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contains a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases. It is also preferred that the transcribed units contain other standard sequences alone or in combination with the above sequences improve expression from, or stability of, the construct.

40. The viral vectors can include nucleic acid sequence encoding a marker product. This marker product is used to determine if the gene has been delivered to the cell and once delivered is being expressed. Preferred marker genes are the *E. Coli* lac*Z* gene, which encodes β-galactosidase, and green fluorescent protein.

41. In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR- cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

42. The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec, Appl. Genet. 1: 327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

43. In one aspect, the disclosed AAV vectors can also comprise a regulatory element (such as, for example the woodchuck posttranscriptional regulatory element (WPRE)) which would not be found in the host endogenously and only present on the first cassette. The regulatory element can be operatively linked to the promoter and/or transgene in the first cassette such that disruption of the regulatory element (such as, through the use of a microRNA, smRNA, RNAi, siRNA, and/or piRNA) turns off expression of the transgene. In one aspect, disruption of the regulator element can be via an RNA silencing element (such as, for example, microRNA, smRNA, RNAi, siRNA, and/or piRNA) which is specific for the regulator element. By linking said RNA silencing element to a tissue specific promoter, expression of the transgene can be prevented from occurring in tissues where expression of the transgene would be deleterious. Thus, in one aspect, disclosed herein are engineered adeno-associated virus (AAV) vectors comprising two expression cassettes; wherein the first cassette comprises a regulatory element (such as, for example WPRE) and a transgene (such as, for example, leptin and/or IL-15) operatively linked to a promoter (such as, for example, the chicken β-actin promoter); and wherein the second cassette comprises a liver specific promoter (such as, for example, the albumin promoter) and a RNA silencing element (such as, for example, microRNA) that targets the regulatory element in the first expression cassette (see Figure 2A); wherein the RNA silencing element is operatively linked to the liver specific promoter.

### Pharmaceutical carriers/Delivery of pharmaceutical products

44. As described above, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

45. The compositions may be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

46. Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795.

47. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

### a) Pharmaceutically Acceptable Carriers

48. The compositions, including antibodies, can be used therapeutically in combination with a pharmaceutically acceptable carrier.

49. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

50. Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

51. Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

52. The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

53. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

54. Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

55. Compositions for oral administration include powders or granules, suspensions or solutions in water or non-aqueous media, capsules, sachets, or tablets Thickeners, flavorings, diluents, emulsifiers, dispersing aids or binders may be desirable.

56. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### b) Therapeutic Uses

57. Effective dosages and schedules for administering the compositions may be determined empirically, and making such determinations is within the skill in the art. The dosage ranges for the administration of the compositions are those large enough to produce the desired effect in which the symptoms of the disorder are effected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any counterindications. Dosage can vary, and can be administered in one or more dose administrations daily, for one or several days. Guidance can be found in the literature for appropriate dosages for given classes of pharmaceutical products. For example, guidance in selecting appropriate doses for antibodies can be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 µg/kg to up to 100 mg/kg of body weight or more per day, depending on the factors mentioned above.

### C. Methods of Treating Obesity, Diabetes, Lipodystrophy, or metabolic syndromes

58. The disclosed AAV vectors can be used for targeted gene disruption, restoration, and modification in any animal that can undergo these events. Gene modification and gene disruption refer to the methods, techniques, and compositions that surround the selective removal, integration, or alteration of a gene or stretch of chromosome in an animal. In general, a cell is transformed with a vector which is designed to homologously recombine with a region of a particular chromosome contained within the cell, as for example, described herein. This homologous recombination event can produce a chromosome which has exogenous DNA introduced, for example in frame, with the surrounding DNA. This type of protocol allows for very specific mutations, to be introduced into the genome contained within the cell.

59. In one aspect, it is understood, that the disclosed vectors can be used to rescue deficiencies of gene/protein expression that lead to a disease. In one aspect, the AAV vectors disclosed herein can be used to treat obesity, diabetes, lipodystrophy (such as, for example, congenital generalized lipodystrophy (also known as Beradinelli-Seip syndrome), familial partial lipodystrophy, acquired partial lipodystrophy (also known as Barraquer-Simons syndrome), acquired generalized lipodystrophy, centrifugal abdominal lipodystrophy, lipoatrophia annularis, localized lipodystrophy, and HIV-associated lipodystrophy), or metabolic syndromes.

60. It is further understood and herein contemplated that activation of NK cell progenitors and NK cells resident in adipose tissue can facilitate treatment of a cancer as the NK cells will attack the cancer. The activation of NK progenitors and NK cells in adipose tissue can be accomplished by providing IL-15 to the NK cells. The engineered AAV vectors disclosed herein being tissue specific can provide this IL-15. Thus, in one aspect, the AAV vectors disclosed herein can be used to treat a cancer (such as, for example, lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, lung cancers such as small cell lung cancer and non-small cell lung cancer, neuroblastoma/glioblastoma, ovarian cancer, pancreatic cancer, prostate cancer, skin cancer, liver cancer, melanoma, squamous cell carcinomas of the mouth, throat, larynx, and lung, colon cancer, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers; testicular cancer; colon cancer, and/or rectal cancer) in a subject by administering an IL-15 encoding AAV vector described herein. Accordingly, in one aspect, disclosed herein are systems for use in methods of treating obesity, diabetes, cancer, lipodystrophy, and/or metabolic syndromes in a subject comprising administering to the subject an engineered adeno-associated virus (AAV) vector comprising two expression cassettes; wherein the first cassette comprises a regulatory element (such as, for example WPRE) and a leptin transgene and/or IL-15 transgene operatively linked to a promoter (such as the CBA promoter); and wherein the second cassette comprises a liver specific promoter (such as the albumin promoter) and a RNA silencing element (such as, for example, microRNA) that targets the regulatory element in the first expression cassette; wherein the RNA silencing element is operatively linked to the liver specific promoter.

61. Also disclosed herein are methods of targeting a nucleic acid based treatment to visceral adipose tissue in a subject comprising administering to the subject an engineered adeno-associated virus (AAV) vector comprising two expression cassettes; wherein the first cassette comprises a regulatory element and a leptin transgene operatively linked to a promoter; and wherein the second cassette comprises a liver specific promoter (such as, for example, albumin) and a RNA silencing element (such as, for example, microRNA) that targets the regulatory element in the first expression cassette; wherein the RNA silencing element is operatively linked to the liver specific promoter; and wherein the AAV vector is administered intraperitoneally.

### D. Examples

62. The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1: Efficient transduction of VAT via IP administration of Rec2 vector

63. To improve gene delivery to VAT, it was firstly determined whether Rec2 vector, through IP administration, a simple and less invasive procedure, could efficiently transduce VAT. Rec2 vector carrying luciferase reporter gene driven by the CMV enhancer and chicken β-actin (CBA) promoter was IP injected to male Balb/c mice at the dose of 2×10¹⁰ vg per mouse that is similar to the doses previously used in IV injection, oral administration, or direct fat pad injection. Two weeks post-Rec2 administration, mice were subjected to *in vivo* bioluminescence measurement and strong luciferase activity was observed primarily in abdomen (Figure 1A). To confirm the efficacy of Rec2 vector via IP administration, Rec2 vectors were generated to deliver another reporter gene GFP and tested three doses (5×10⁸, 2×10⁹, 1×10¹⁰ vg per mouse). Two weeks post Rec2 injection, tissues were harvested and GFP fluorescence was measured in fresh tissue lysates. Rec2-GFP at the dose of 1×10¹⁰ vg per mouse resulted in robust GFP expression in all visceral fat depots examined including epididymal (eWAT), mesenteric (mWAT), and retroperitoneal (rWAT) (Figure 1B). GFP fluorescence was also detected in liver but not in intestine (Figure 1B). Immunohistochemistry confirmed the transgene expression in VAT and liver (Figure 1C).

### 2. Example 2: Preferential transduction of VAT using a dual-cassette Rec2 vector restricting off-target transgene expression in liver

64. To prevent transgene expression in the liver, a new AAV expression plasmid was generated that contained two expression cassettes: one used CBA promoter to drive the transgene, and the other used the liver-specific albumin promoter to drive a specific microRNA targeting woodchuck posttranscriptional regulatory element (WPRE) sequence which only exists in the transgene expression cassette (Figure 2A). WPRE enhances transgene expression and is incorporated in all of the AAV expression plasmids. The microRNA targeting WPRE (miR-WPRE) knocked down transgene expression more than 90% *in vitro* as measured by quantitative RT-PCR and ELISA. The efficacy of this dual-cassettes vector (named AS/Rec2) was assessed by IP injection of AS/Rec2-GFP to wild type mice. Incorporation of the liver-restricting cassette severely decreased transgene expression in liver at the higher dose of 2×10¹⁰ vg per mouse (Figure 2B). At lower dose of 1×10¹⁰ vg per mouse, GFP fluorescence was undetectable in liver while substantial GFP fluorescence was maintained in eWAT (Figure 2B). To further characterize the AS/Rec2 vector, a dose-response experiment was performed with four doses: 1×10¹⁰, 2×10¹⁰, 3×10¹⁰, and 4×10¹⁰ vg per mouse. Across the range of doses, hepatic transgene expression was severely suppressed even though large amount of viral vector DNA was detected in liver (Figure 2C, D) confirming the efficacy of the dual-cassette vector. GFP fluorescence was undetectable in small intestine, large intestine, spleen, kidney, testis, brown fat, or subcutaneous fat.

### 3. Example 3: Molecular therapy of congenital leptin deficiency via IP administration of AS/Rec2-leptin vector

65. To investigate the therapeutic potential of the AS/Rec2 vector, AS/Rec2-leptin vector was generated to treat leptin-deficient obesity model *ob*/*ob* mice with AS/Rec2-GFP as control. Male *ob*/*ob* mice, 6 weeks of age, were randomized to receive IP injection of AS/Rec2-leptin or AS/Rec2-GFP, 4×10¹⁰ vg per mouse. *ob*/*ob* mice at 6 weeks of age were obese compared to age-matched wild type (WT) mice. AS/Rec2-GFP-treated *ob*/*ob* mice continued to gain weight rapidly and become morbid obese (Figure 3A, B, C). In contrast, AS/Rec2-leptin treatment completely prevented weight gain and normalized body weight close to age-matched WT mice 4 weeks post AS/Rec2-leptin injection (Figure 3A-C, Figure 4C). The weight loss was stable and sustained throughout the entire 9-week duration of the study. A sharp drop of food intake was observed in AS/Rec2-leptin-treated mice as early as 1-week post AAV administration and the reversal of hyperphagia of *ob*/*ob* mice were maintained similarly to the weight loss (Figure 3D). AS/Rec2-leptin treatment also corrected the abnormal low body temperature of *ob*/*ob* mice and impaired thermogenesis in response to fast (Figure 3E). Four weeks post-Rec2 administration, body composition was determined using EchoMRI. Adiposity was decreased by 65% while lean mass was increased by 75% in AS/Rec2-leptin-treated mice compared to GFP mice (Figure 3F). Serum leptin level was measured using ELISA 4-week post AAV administration. AS/Rec2-leptin treatment resulted in circulating leptin level slightly lower than age-matched WT mice with no statistical significance (Figure 4A) while leptin was undetectable in GFP-treated *ob*/*ob* mice. AS/Rec2-leptin treatment completely rescued the impaired glycemic control of *ob*/*ob* mice (Figure 4B). In fact, blood glucose level in AS/Rec2-leptin-treated *ob*/*ob* mice was even lower than WT mice at a few time points of the glucose tolerance test (Figure 4B). Indirect calorimetry was performed 5 weeks after Rec2 administration. AS/Rec2-leptin-treated mice showed increased oxygen consumption and locomotor activity in both the dark and light phases (Figure 4E, F) consistent with elevated energy expenditure. In contrast, food intake was lower in AS/Rec2-leptin-treated mice (Figure 4D) similar to the findings in home cage (Figure 3D).

66. At sacrifice 9 weeks after AAV injection, eWAT of AS/Rec2-leptin-treated mice was decreased by 82.5% compared to GFP-treated mice when calibrated to body weight (Figure 5A). Liver mass was also decreased (Figure 5A) and the liver steatosis was completely reversed by AS/Rec2-leptin treatment (Figure 5C). Moreover, AS/Rec2-leptin treatment completely corrected the hyperinsulinemia of *ob*/*ob* mice (Figure 5B). Transgene expression in visceral fat depots was confirmed in AS/Rec2-GFP mice whereas minimal GFP fluorescence was detected in the livers (Figure 5D).

### 4. Example 4: DISCUSSION

67. It is challenging to adapt traditional techniques to selectively modulate adipose functions *in vivo* for experimental and therapeutic applications. A few groups have targeted adipose tissue with naturally occurring AAV serotypes by direct injection to fat depots with limited success. An engineered hybrid serotype Rec2 displays superior transduction of both BAT and subcutaneous WAT (SAT) compared to AAVI or AAV8. Direct injection of Rec2 vector at a moderate dose (1×10⁹ to 1×10¹⁰ vg per fat depot) is sufficient to modulate the function of the targeted fat depot (BAT or SAT) and result in systemic metabolic changes. Rec2 vector is efficient for both overexpression and knockdown applications. However, up to date, scarce studies report systemic administration of AAV to transduce multiple adipose depots. One study reports that intravenous administration of AAV8 vector transduces adipose tissues, liver and other tissues. Replacing the CMV promoter with human adiponectin enhancer/promoter enhances specificity to adipose tissue whereas transgene expression is not suppressed in liver. Incorporation of miR-122 target sequence (miR-122T) into the 3'UTR of AAV vector can eliminate hepatic transgene expression, but often at the expense of lower transgene expression in adipose tissue. In addition, the dose of 1×10¹² vg per mouse used in this study is approximately 100 fold higher than the dose of Rec2 vector direct fat injection. In this work, a novel dual-cassette AAV expression plasmid was designed and combined this liver-restricting strategy with the adipo-trophic Rec2 serotype and IP administration to achieve, for the first time, selective transduction of multiple visceral fat depots. More encouraging is the low dose for IP administration of this new AAV vector system that is equivalent to direct fat injection and 2 orders lower than the reported IV administration of AAV8 based adipose-targeting system.

68. To enhance tissue-preferential transduction particularly when using systemic administration, miR-122T is often used to suppress off-target transgene expression in liver because miR-122 is highly abundant in liver. However, this approach can cause toxicities, because exogenous miR-122T inside a highly expressed hepatocyte can compete with endogenous miR-122T for miR-122. And liver toxicities including hepatic steatosis, hepatitis, or hepatocellular carcinoma have been reported in miR-122 knockout mice. Although this risk is very low, an alternative approach to eliminate the risk improves safety for clinical applications. A liver-specific albumin promoter was used to drive a microRNA targeting WPRE that only existed in the same AAV vector harboring the transgene, and therefore does not interfere with any endogenous genes or microRNAs. The miR-WPRE was highly efficient to knockdown the transgene expression even being driven by a strong CBA promoter, and in combination with a proper dose, sufficient transgene expression in VAT whereas minimal hepatic expression was achieved.

69. An interesting characteristic of Rec2 serotype is that its tissue tropism is influenced by administration route. Oral administration leads to preferential transduction of distal interscapular BAT with minimal transduction of stomach or intestine. Intravenous injection results in transduction primarily in liver. IP injection of Rec2 robustly transduced VAT whereas minimal transgene expression observed in SAT or BAT. This feature allows preferential modulation of VAT, the fat depots closely associated with risk of metabolic syndromes and heart disease. Furthermore, IP administration of Rec2 vector enables genetic manipulation of mWAT that is otherwise difficult to access even via invasive surgical approaches.

70. Obesity has a substantial genetic component. Congenital leptin deficiencies in humans cause severe obesity, hyperphagia, and hyperinsulinemia. Leptin replacement therapy is necessary in patients with homozygous Lep mutations or acquired leptin deficiency derived from congenital or acquired lipodystrophy. Treatment with recombinant leptin therapy through subcutaneous injection produces short lasting effects, and therefore requires repetitive doses. And the supra-physiological increase in circulating leptin level following regular injection is associated with serious side effects. Gene therapies with AAV vectors have been attempted in the *ob*/*ob* mice, an animal model best recapitulating human congenital leptin deficiencies. AAV-mediated intramuscular leptin gene transfer (1×10¹¹ vg per injection) alleviates obesity and diabetes. Up to date, only one gene therapy study is reported to reintroduce the leptin gene in its native tissue. Intravenous injection of AAV2/8 based adipose-targeting vector to *ob*/*ob* mice at the dose of 1×10¹² vg per mouse, results in peak leptin level approximately 7% of circulating leptin level in the age-matched WT mice and partially attenuates the metabolic syndromes. In contrast, in the same *ob*/*ob* mouse model, IP injection of the novel adipose-targeting Rec2 vector at 4×10¹⁰ vg per mouse, 25 fold lower than the reported dose, normalized leptin level close to the age-matched WT (Figure 4A). Moreover, AS/Rec2-leptin treatment completely reversed hyperinsulinemia and impaired glucose tolerance, and robustly corrected other metabolic symptoms including obesity, hyperphagia, low energy expenditure, impaired thermogenesis, and low physical activity much closer to the age-matched WT animals, indicating advances compared to the limited efficacy with previously reported AAV systems.

71. Both animal and human studies have demonstrated that restoring circulating leptin level to 10% of normal levels are sufficient to alleviate metabolic syndromes associated with congenital leptin deficiency. Thus it is likely that the dose of AS/Rec-leptin could be lowered further to 1× 10¹⁰ vg per mouse, a dose based on body mass near the 1×10¹² vg per kg dose of alipogene tiparvovec (Glybeta) that has been demonstrated to be safe and effective in patients. Moreover, clinical studies of genetic deficiencies in factor VIII, factor IX hemophilia, and lipoprotein lipase (LPL) have shown that AAV gene therapy resulting in very small corrections of physiological levels (5 to 10% of normal levels) is able to profoundly reverse physiologic aberrancies including the first approved gene therapy drug in Europe. Adipose tissue is a secretory organ and mature adipocytes are terminally differentiated and non-dividing, and therefore an attractive target for non-integrating gene expression vectors such as AAV. The high efficiency of the new adipose-targeting AAV vector indicates potentially wider applications, for example, replacing a defective gene in adipose tissues, inducing adipose phenotypic changes to achieve systemic benefits, or producing therapeutic proteins for diseases not associated with adipose dysfunction.

72. In summary, the data demonstrate, for the first time, that IP administration of a novel AAV vector selectively transduces visceral fat depots with minimal off-target transgene expression in liver. One administration of AS/Rec2-leptin at a dose much lower than previous report lead to sustained and near-complete reversal of metabolic abnormalities in a mouse model representing a human genetic disease, congenital leptin deficiency.

### 5. Example 5: MATERIALS and METHODS

### (1) Mice

73. Wild type C57/BL6 mice, 8 to 12 weeks of age, were purchased from Charles River (Spencerville, OH). *ob*/*ob* mice on a C57/BL6 background were purchased from Jackson Laboratories (Bar Harbor, ME). All mice were housed in a temperature-controlled room (22-23 °C) with a 12 h light-12 h dark cycle, and maintained on a standard rodent diet (7912 rodent chow, Teklad) with free access to food and water *ad libitum.* Male mice were used in all studies. All use of animals was approved by, and in accordance with the Ohio State University Animal Care and Use Committee.

### (2) AAV vector construction and package

74. The rAAV plasmid contains a vector expression cassette consisting of the CMV enhancer and chicken β-actin (CBA) promoter, woodchuck post-transcriptional regulatory element (WPRE) and bovine growth hormone (bGH) poly-A flanked by AAV2 inverted terminal repeats. Transgenes, e.g. GFP, luciferase, were inserted into the multiple cloning sites between the CBA promoter and WPRE sequence. Rec2 vectors were packaged and purified.

### (3) microRNA targeting WPRE.

75. Two targeting sequences in the WPRE sequence were cloned into the Block-iT PolII miR RNAi expression vector (pcDNA6.2-Gw/miR, Invitrogen). The knockdown efficiency was determined by co-transfecting HEK293 cells with a standard AAV expression plasmid containing BDNF as the transgene. In *in vitro* experiments, both miR constructs inhibited BDNF expression by at least 90% confirmed by qRT-PCR and BDNF ELISA (Promega). The miR-WPRE (mature miR seq: CTATGTGGACGCTGCTTTA) (SEQ ID NO: 1) was chosen for the construction of dual-cassette plasmid.

### (4) Dual-cassette adipose-specific vector construction and package

76. To construct dual-cassette adipose-specific expression plasmid, proximal region of mouse albumin promoter (-317 to +18, the cap site is designated as 1) was generated by using pALB-GFP as a template with XbaI and BamHI flanking at each side. pALB-GFP is a gift from Snorri Thorgeirsson (Addgene plasmid #55759). The liver specific albumin promoter length is based on A 400 bp fragment of rat albumin gene immediate 5'-flanking sequences spanning the -390 to 10 bp leads to reporter gene expression only in cell lines which express the rat albumin This 400 bp sequence is considered as a highly cell-specific promoter. We chose the mouse albumin promoter sequence based on the homology between mouse and rat albumin genes ]. Mouse leptin cDNA was amplified from mouse adipose tissue cDNA, sequenced and then subcloned into the multiple cloning sites between CBA promoter and WPRE. The cassette containing miR-WPRE driven by albumin promoter was then cloned to the AAV expression plasmid containing transgenes (leptin or GFP) to generate dual-cassette plasmids (Fig 2). Rec2 serotype vectors were packaged as described above.

### (5) Administration of Rec2 vector

77. Rec2 vectors were administered into mice through intraperitoneal injection in 150 µl AAV dilution buffer. For wild type mice experiments, doses ranging from 5×10⁹ to 4×10¹⁰ vg per mouse were used. For *ob*/*ob* mice experiment, AS/Rec2-leptin vector at a dose of 4×10¹⁰ vg per mouse was IP injected.

### (6) Luciferase imaging

78. The luciferase imaging was carried out two weeks after delivery of Rec2-luciferase via IP administration (2×10¹⁰ vg per mouse). The hairs over abdomen were partially removed. D-luciferin, potassium salt (Gold Biotechnology, Inc.) was intraperitoneally injected into each mouse at a dose of 150 µg/g body weight. After ten minutes, the mice were anesthetized in isoflurane chamber and then placed in a warm- and light-tight scan chamber. The bioluminescence imaging was generated using IVIS Lumina II, Caliper (Small Animal Imaging Core Facility of OSU) and presented by radiance unit of photons/second/cm²/sr, which is the number of photons per second that leave a square centimeter of tissue and radiate into a solid angle of one steradian (sr).

### (7) GFP content measurement

**79.** Fat pads were dissected. GFP content was measured. In brief, tissues were homogenized in ice-cold RIPA buffer (25mM Tris-HCl pH 7.6, 150mM NaCl, 1% NP-40, 1% sodium deoxycholate, 0.1% SDS) supplemented with proteinase inhibitors cocktail, followed by briefly sonication. The mixture was then spun at 13,000 rpm for 15 min at 4 °C. The supernatant was collected. GFP fluorescence in 100 µl supernatant was measured with microreader (Texas Instrument) using a 488-nm excitation wavelength and cut-off 515-nm/525-nm emission wavelength. GFP content was represented as readings after subtracting auto-fluorescence from GFP-null corresponding tissue and corrected by protein content in the samples.

### (8) ob/ob mice experiment

80. Six weeks old *ob*/*ob* mice were randomly assigned to receive AS/Rec2-leptin or AS/Rec2-GFP (4×10¹⁰ vg per mouse, IP). Body weight and food intake were monitored weekly over the period of 9 weeks.

### (9) Glucose tolerance test

81. Four weeks after IP administration of Rec2 vectors, mice were IP injected with glucose solution (1mg glucose per g body weight) after overnight fast. Blood was drawn from the tail at various time points and the blood glucose concentrations were measured with a portable glucose meter (Bayer Contour Next).

### (10) Recta temperature measurement

82. Temperature probe (Physitemp, model BAT-12) topped with lubricant (white petroleum jelly) was used to measure the rectal temperature of *ob*/*ob* mice at various time points after IP administration of Rec2 vectors.

### (11) Metabolic studies

83. Five weeks after IP administration of Rec2 vectors, *ob*/*ob* mice were subjected to indirect calorimetry using the Oxymax Lab Animal Monitoring System (Columbus Instruments). Individual mouse was habituated to the instrument for 24 h, and the physiological and behavioral parameters were monitored for 48 h (activity, food and water consumption, metabolic performance and temperature). Oxygen consumption, carbon dioxide production and methane production were normalized to the body weight and corrected to an effective mass value according to the manufacturer's software.

### (12) Body composition by EchoMRI

84. EchoMRI was used to measure body composition of fat, lean, free water, and total water masses in live mice without anesthesia. EchoMRI imaging was performed with EchoMRI Analyzer at Small Animal Imaging Core of The Dorothy M. Davis Heart & Lung Research Institute, Ohio State University.

### (13) Histology and immunohistochemistry

85. Liver tissues were frozen in OCT and cryo-sectioned at 8 µm. Lipids in liver were stained on frozen sections with an Oil Red O solution (Sigma). Adipose tissues were fixed in 10% formalin, then embedded in paraffin and sectioned at 5 µm at Core Facility of OSU Comprehensive Cancer Center. Paraffin-embedded sections were subjected to citrate-based antigen retrieval followed by incubation with antibody against GFP (Abcam, Cambridge). The sections were visualized with 3,3 Diaminobenzidine (DAB) and counterstained with hematoxylin.

### (14) Metabolic parameters

86. Serum leptin was measured using a DuoSet ELISA Development System (R&D Systems). Serum insulin was determined with mouse insulin ELISA (ALPCO).

### (15) Viral vector copy number measurement

87. Total DNA from tissues was isolated using DNesay Blood and Tissue kit (Qiagen). WPRE fragment was amplified to determine the copy number of viral particle. 50 ng of DNA from each sample was used for real time PCR. Mouse nucleic genomic fragment of GAPDH gene was used as control for mouse genetic DNA. The standard curve for copy number was generated from a known plasmid DNA. Viral vectors were isolated and purified from blood using High Pure Viral Nucleic Acid Kit (Roche).

### (16) Statistical analysis

88. Data are expressed as mean±SEM. JMP software was used to analyze the following: two-way analysis of variance for body weight, food intake, and glucose tolerance; Student's *t* test for adiposity, body temperature, and serum biomarkers data.

### E. References

Agostini, M., Schoenmakers, E., Mitchell, C., Szatmari, I., Savage, D., Smith, A., Rajanayagam, O., Semple, R, Luan, J., Bath, L., et al. (2006). Non-DNA binding, dominant-negative, human PPARgamma mutations cause lipodystrophic insulin resistance. Cell Metab 4, 303-311.

Arruda, V.R., Stedman, H.H., Nichols, T.C., Haskins, M.E., Nicholson, M., Herzog, R.W., Couto, L.B., and High, K.A. (2005). Regional intravascular delivery of AAV-2-F.IX to skeletal muscle achieves long-term correction of hemophilia B in a large animal model. Blood 105, 3458-3464.

Baldo, B.A. (2014). Side effects of cytokines approved for therapy. Drug Saf 37, 921-943.

Barsh, G.S., Farooqi, I.S., and O'Rahilly, S. (2000). Genetics of body-weight regulation. Nature 404, 644-651.

Bray, G.A. (1991). Obesity, a disorder of nutrient partitioning: the MONA LISA hypothesis. J Nutr 121, 1146-1162.

Brown, B.D., Gentner, B., Cantore, A., Colleoni, S., Amendola, M., Zingale, A., Baccarini, A., Lazzari, G., Galli, C., and Naldini, L. (2007). Endogenous microRNA can be broadly exploited to regulate transgene expression according to tissue, lineage and differentiation state. Nat Biotechnol 25, 1457-1467.

Cao, H., Molday, R.S., and Hu, J. (2011). Gene therapy: light is finally in the tunnel. Protein Cell 2, 973-989.

Cao, L., Jiao, X., Zuzga, D.S., Liu, Y., Fong, D.M., Young, D., and During, M.J. (2004). VEGF links hippocampal activity with neurogenesis, learning and memory. Nature genetics 36, 827-835.

Chau, Y.Y., Bandiera, R., Serrels, A., Martinez-Estrada, O.M., Qing, W., Lee, M., Slight, J., Thombum, A., Berry, R., McHaffie, S., et al. (2014). Visceral and subcutaneous fat have different origins and evidence supports a mesothelial source. Nat Cell Biol 16, 367-375.

Cortes, V.A., Curtis, D.E., Sukumaran, S., Shao, X., Parameswara, V., Rashid, S., Smith, A.R., Ren, J., Esser, V., Hammer, R.E., et al. (2009). Molecular mechanisms of hepatic steatosis and insulin resistance in the AGPAT2-deficient mouse model of congenital generalized lipodystrophy. Cell Metab 9, 165-176,

During, M.J., Liu, X., Huang, W., Magee, D., Slater, A., McMurphy, T., Wang, C., and Cao, L. (2015). Adipose VEGF Links the White-to-Brown Fat Switch With Environmental, Genetic, and Pharmacological Stimuli in Male Mice. Endocrinology 156, 2059-2073.

Esau, C., Davis, S., Murray, S.F., Yu, X.X., Pandey, S.K., Pear, M., Watts, L., Booten, S.L., Graham, M., McKay, R., et al. (2006). miR-122 regulation of lipid metabolism revealed by in vivo antisense targeting. Cell Metab 3, 87-98.

Farooqi, I.S., Jebb, S.A., Langmack, G., Lawrence, E., Cheetham, C.H., Prentice, A.M., Hughes, I.A., McCamish, M.A., and O'Rahilly, S. (1999). Effects of recombinant leptin therapy in a child with congenital leptin deficiency. N Engl J Med 341, 879-884.

Farooqi, I.S., Matarese, G., Lord, G.M., Keogh, J.M., Lawrence, E., Agwu, C., Sanna, V., Jebb, S.A., Perna, F., Fontana, S., et al. (2002). Beneficial effects of leptin on obesity, T cell hyporesponsiveness, and neuroendocrine/metabolic dysfunction of human congenital leptin deficiency. The Journal of clinical investigation 110, 1093-1103.

Friedman, J. (2016). The long road to leptin. The Journal of clinical investigation 126, 4727-4734.

Gaudet, D., Methot, J., Dery, S., Brisson, D., Essiembre, C., Tremblay, G., Tremblay, K., de Wal, J., Twisk, J., van den Bulk, N., et al. (2013). Efficacy and long-term safety of alipogene tiparvovec (AAV1-LPLS447X) gene therapy for lipoprotein lipase deficiency: an open-label trial. Gene therapy 20, 361-369.

Geisler, A., Jungmann, A., Kurreck, J., Poller, W., Katus, H.A., Vetter, R., Fechner, H., and Muller, O.J. (2011). microRNA122-regulated transgene expression increases specificity of cardiac gene transfer upon intravenous delivery of AAV9 vectors. Gene therapy 18, 199-209.

Gibson, W.T., Farooqi, I.S., Moreau, M., DePaoli, A.M., Lawrence, E., O'Rahilly, S., and Trussell, R.A. (2004). Congenital leptin deficiency due to homozygosity for the Delta133G mutation: report of another case and evaluation of response to four years of leptin therapy. J Clin Endocrinol Metab 89, 4821-4826.

Gorski, K., Carneiro, M., and Schibler, U. (1986). Tissue-specific in vitro transcription from the mouse albumin promoter. Cell 47, 767-776.

Heo, J., Factor, V.M., Uren, T., Takahama, Y., Lee, J.S., Major, M., Feinstone, S.M., and Thorgeirsson, S.S. (2006). Hepatic precursors derived from murine embryonic stem cells contribute to regeneration of injured liver. Hepatology 44, 1478-1486.

Huang, W., McMurphy, T., Liu, X., Wang, C., and Cao, L. (2016). Genetic Manipulation of Brown Fat Via Oral Administration of an Engineered Recombinant Adeno-associated Viral Serotype Vector. Molecular therapy : the journal of the American Society of Gene Therapy 24, 1062-1069.

Ibrahim, M.M. (2010). Subcutaneous and visceral adipose tissue: structural and functional differences. Obes Rev 11, 11-18.

Ingalls, A.M., Dickie, M.M., and Snell, G.D. (1950). Obese, a new mutation in the house mouse. J Hered 41, 317-318.

Jimenez, V., Munoz, S., Casana, E., Mallol, C., Elias, I., Jambrina, C., Ribera, A., Ferre, T., Franckhauser, S., and Bosch, F. (2013). In vivo adeno-associated viral vector-mediated genetic engineering of white and brown adipose tissue in adult mice. Diabetes 62, 4012-4022.

Kotterman, M.A., and Schaffer, D.V. (2014). Engineering adeno-associated viruses for clinical gene therapy. Nature reviews Genetics 15, 445-451.

Lafontan, M., and Berlan, M. (2003). Do regional differences in adipocyte biology provide new pathophysiological insights? Trends Pharmacol Sci 24, 276-283.

Liu, X., Magee, D., Wang, C., McMurphy, T., Slater, A., During, M., and Cao, L. (2014). Adipose tissue insulin receptor knockdown via a new primate-derived hybrid recombinant AAV serotype. Molecular therapy Methods & clinical development 1.

Liu, X.M., D.; Wang, C. McMurthpy, T.; Slater, A., During, M.J.; Cao, L. (2014). Adipose tissue insulin receptor knockdown via a new primate-derived hybrid recombinant AAV serotype. Molecular Therapy-Methods & Clinical Development 1.

Loeb, J.E., Cordier, W.S., Harris, M.E., Weitzman, M.D., and Hope, T.J. (1999). Enhanced expression of transgenes from adeno-associated virus vectors with the woodchuck hepatitis virus posttranscriptional regulatory element: implications for gene therapy. Human gene therapy 10, 2295-2305.

Maffei, M., Halaas, J., Ravussin, E., Pratley, R.E., Lee, G.H., Zhang, Y., Fei, H., Kim, S., Lallone, R., Ranganathan, S., et al. (1995). Leptin levels in human and rodent: measurement of plasma leptin and ob RNA in obese and weight-reduced subjects. Nat Med 1, 1155-1161.

Mann, A., Thompson, A., Robbins, N., and Blomkalns, A.L. (2014). Localization, identification, and excision of murine adipose depots. J Vis Exp.

McMurphy, T.B., Huang, W., Xiao, R., Liu, X., Dhurandhar, N.V., and Cao, L. (2017). Hepatic Expression of Adenovirus 36 E4ORF1 Improves Glycemic Control and Promotes Glucose Metabolism Through AKT Activation. Diabetes 66, 358-371.

Mingozzi, F., and High, K.A. (2011). Therapeutic in vivo gene transfer for genetic disease using AAV: progress and challenges. Nature reviews Genetics 12, 341-355.

Mizukami, H., Mimuro, J., Ogura, T., Okada, T., Urabe, M., Kume, A., Sakata, Y., and Ozawa, K. (2006). Adipose tissue as a novel target for in vivo gene transfer by adeno-associated viral vectors. Human gene therapy 17, 921-928.

Montague, C.T., Farooqi, I.S., Whitehead, J.P., Soos, M.A., Rau, H., Wareham, N.J., Sewter, C.P., Digby, J.E., Mohammed, S.N., Hurst, J.A., et al. (1997). Congenital leptin deficiency is associated with severe early-onset obesity in humans. Nature 387, 903-908.

Murphy, J.E., Zhou, S., Giese, K., Williams, L.T., Escobedo, J.A., and Dwarki, V.J. (1997). Long-term correction of obesity and diabetes in genetically obese mice by a single intramuscular injection of recombinant adeno-associated virus encoding mouse leptin. Proc Natl Acad Sci U S A 94, 13921-13926.

O'Neill, S.M., Hinkle, C., Chen, S.J., Sandhu, A., Hovhannisyan, R., Stephan, S., Lagor, W.R., Ahima, R.S., Johnston, J.C., and Reilly, M.P. (2014). Targeting adipose tissue via systemic gene therapy. Gene therapy 21, 653-661.

Oral, E.A., Simha, V., Ruiz, E., Andewelt, A., Premkumar, A., Snell, P., Wagner, A.J., DePaoli, A.M., Reitman, M.L., Taylor, S.I., et al. (2002). Leptin-replacement therapy for lipodystrophy. N Engl J Med 346, 570-578.

Ott, MO., Sperling, L., Herbomel, P., Yaniv, M., and Weiss, M.C. (1984). Tissue-specific expression is conferred by a sequence from the 5' end of the rat albumin gene. EMBO J 3, 2505-2510.

Peyvandi, F., Palla, R., Menegatti, M., Siboni, S.M., Halimeh, S., Faeser, B., Pergantou, H., Platokouki, H., Giangrande, P., Peerlinck, K., et al. (2012). Coagulation factor activity and clinical bleeding severity in rare bleeding disorders: results from the European Network of Rare Bleeding Disorders. J Thromb Haemost 10, 615-621.

Qiao, C., Yuan, Z., Li, J., He, B., Zheng, H., Mayer, C., Li, J., and Xiao, X. (2011). Liver-specific microRNA-122 target sequences incorporated in AAV vectors efficiently inhibits transgene expression in the liver. Gene therapy 18, 403-410.

Tronche, F., Rollier, A., Bach, I., Weiss, M.C., and Yaniv, M. (1989). The rat albumin promoter: cooperation with upstream elements is required when binding of APF/HNF1 to the proximal element is partially impaired by mutation or bacterial methylation. Mol Cell Biol 9, 4759-4766.

Wen, J., and Friedman, J.R. (2012). miR-122 regulates hepatic lipid metabolism and tumor suppression. The Journal of clinical investigation 122, 2773-2776.

Wooddell, C.I., Reppen, T., Wolff, J.A., and Herweijer, H. (2008). Sustained liver-specific transgene expression from the albumin promoter in mice following hydrodynamic plasmid DNA delivery. J Gene Med 10, 551-563.

Yla-Herttuala, S. (2012). Endgame: glybera finally recommended for approval as the first gene therapy drug in the European union. Molecular therapy : the journal of the American Society of Gene Therapy 20, 1831-1832.

Zhang, F.L., Jia, S.Q., Zheng, S.P., and Ding, W. (2011). Celastrol enhances AAV1-mediated gene expression in mice adipose tissues. Gene therapy 18, 128-134.

Zhu, Y., Gao, Y., Tao, C., Shao, M., Zhao, S., Huang, W., Yao, T., Johnson, J.A., Liu, T., Cypess, A.M., et al. (2016). Connexin 43 Mediates White Adipose Tissue Beiging by Facilitating the Propagation of Sympathetic Neuronal Signals. Cell Metab 24, 420-433.

Zufferey, R., Donello, J.E., Trono, D., and Hope, T.J. (1999). Woodchuck hepatitis virus posttranscriptional regulatory element enhances expression of transgenes delivered by retroviral vectors. J Virol 73, 2886-2892.

## Claims

1. A system for delivering a transgene to visceral adipose tissue comprising a hybrid serotype Rec2 engineered adeno-associated virus (AAV) vector comprising two expression cassettes; wherein the first cassette comprises a regulatory element and a transgene operatively linked to a promoter; and wherein the second cassette comprises a tissue specific promoter operatively linked to a RNA silencing element that targets the regulatory element in the first expression cassette.

2. The system of claim 1, wherein the regulatory element of the first cassette is a woodchuck posttranscriptional regulatory element (WPRE) sequence.

3. The system of claim 1 or 2, wherein the transgene of the first cassette is a leptin gene.

4. The system of claim 1 or 2, wherein the transgene of the first cassette is a IL-15 gene.

5. The system of any of claims 1 to 4, wherein the RNA silencing element is a microRNA.

6. The system of any of claims 1-5, wherein the tissue specific promoter of the second cassette is a liver specific promoter.

7. The system of claim 6, wherein the liver specific promoter is an albumin promoter, thyroxine binding globin promoter, phosphoenolpyruvatecarboxykinase (PEPCK) promoter, tyrosine aminotransgerase (TAT) promoter, or human alpha 1-antitrypsin (hAAT) promoter.

8. The system of any one of the preceding claims, for use in therapy.

9. The system of any one of the preceding claims for use in a method of treating diabetes, obesity, metabolic syndrome, or lipodystrophy in a subject; wherein the first cassette comprises a regulatory element and a leptin transgene operatively linked to a promoter; and wherein the second cassette comprises a liver specific promoter operatively linked to a microRNA that targets the regulatory element in the first expression cassette.

10. The system of any one of claims 1 to 8 for use in a method of treating cancer in a subject; wherein the first cassette comprises a regulatory element and an IL-15 transgene operatively linked to a promoter; and wherein the second cassette comprises a liver specific promoter operatively linked to a microRNA that targets the regulatory element in the first expression cassette.

11. The system of claim 9 or 10, wherein the regulatory element of the first cassette is a woodchuck posttranscriptional regulatory element (WPRE) sequence.

12. The system according to any one of claims 8 to 11, wherein the AAV vector is administered intraperitoneally.

13. The system of one of claims 9 to 12, wherein the liver specific promoter is an albumin promoter, thyroxine binding globin promoter, phosphoenolpyruvatecarboxykinase (PEPCK) promoter, tyrosine aminotransgerase (TAT) promoter, or human alpha 1-antitrypsin (hAAT) promoter.

## Patentansprüche

1. System zur Abgabe eines Transgens an viszeral adipöses Gewebe, umfassend einen hybrid Serotyp Rec2-veränderten Adeno-assoziierten Virus (AAV)-Vektor, umfassend zwei Expressionskassetten; wobei die erste Kassette ein regulatorisches Element und ein Transgen umfasst, das operativ mit einem Promotor verbunden ist; und wobei die zweite Kassette einen gewebespezifischen Promotor umfasst, der operativ mit einem RNA Stummschaltungselement verbunden ist, das auf das regulatorische Element in der ersten Expressionskassette abzielt.

2. System nach Anspruch 1, wobei das regulatorische Element der ersten Kassette eine Woodchuck Posttranscriptional Regulatory Element (WPRE)-Sequenz ist.

3. System nach Anspruch 1 oder 2, wobei das Transgen der ersten Kassette ein Leptingen ist.

4. System nach Anspruch 1 oder 2, wobei das Transgen der ersten Kassette ein IL-15 Gen ist.

5. System nach einem der Ansprüche 1 bis 4, wobei das RNA Stummschaltungselement eine microRNA ist.

6. System nach einem der Ansprüche 1 bis 5, wobei der gewebespezifische Promotor der zweiten Kassette ein leberspezifischer Promotor ist.

7. System nach Anspruch 6, wobei der leberspezifische Promotor ein Albumin-Promotor, thyroxinbindender Globin-Promotor, Phosphoenolpyruvatcarboxykinase (PEPCK)-Promotor, Tyrosinaminotransgerase (TAT)-Promotor oder humaner alpha-1-Antitrypsin (hAAT)-Promotor ist.

8. System nach einem der vorhergehenden Ansprüche, zur Verwendung in der Therapie.

9. System nach einem der vorhergehenden Ansprüche zur Verwendung in einem Verfahren zur Behandlung von Diabetes, Adipositas, metabolischem Syndrom oder Lipodystrophie bei einem Patienten; wobei die erste Kassette ein regulatorisches Element und ein Leptin-Transgen umfasst, das operativ mit einem Promotor verbunden ist; und wobei die zweite Kassette einen leberspezifischen Promotor umfasst, der operativ mit einer microRNA verbunden ist, die auf das regulatorische Element in der ersten Expressionskassette abzielt.

10. System nach einem der Ansprüche 1 bis 8 zur Verwendung in einem Verfahren zur Behandlung von Krebs bei einem Patienten; wobei die erste Kassette ein regulatorisches Element und ein IL 15 Transgen umfasst, das operativ mit einem Promotor verbunden ist; und wobei die zweite Kassette einen leberspezifischen Promotor umfasst, der operativ mit einer microRNA verbunden ist, die auf das regulatorische Element in der ersten Expressionskassette abzielt.

11. System nach Anspruch 9 oder 10, wobei das regulatorische Element der ersten Kassette eine Woodchuck Posttranscriptional Regulatory Element (WPRE)-Sequenz ist.

12. System nach einem der Ansprüche 8 bis 11, wobei der AAV-Vektor intraperitoneal verabreicht wird.

13. System nach einem der Ansprüche 9 bis 12, wobei der leberspezifische Promotor ein Albumin-Promotor, thyroxinbindender Globin-Promotor, Phosphoenolpyruvatcarboxykinase (PEPCK)-Promotor, Tyrosinaminotransgerase (TAT)-Promotor oder humaner alpha-1-Antitrypsin (hAAT)-Promotor ist.

## Revendications

1. Système pour administrer un transgène à un tissu adipeux viscéral comprenant un vecteur viral adéno-associé (AAV) modifié de sérotype Rec2 hybride comprenant deux cassettes d'expression ; dans lequel la première cassette comprend un élément régulateur et un transgène lié de manière fonctionnelle à un promoteur ; et dans lequel la deuxième cassette comprend un promoteur spécifique d'un tissu lié de manière fonctionnelle à un élément interfèrent de type ARN qui cible l'élément régulateur dans la première cassette d'expression.

2. Système selon la revendication 1, dans lequel l'élément régulateur de la première cassette est une séquence d'élément régulateur post-transcription de marmotte (WPRE).

3. Système selon la revendication 1 ou 2, dans lequel le transgène de la première cassette est un gène de leptine.

4. Système selon la revendication 1 ou 2, dans lequel le transgène de la première cassette est un gène d'IL-15.

5. Système selon l'une quelconque des revendications 1 à 4, dans lequel l'élément interfèrent de type ARN est un microARN.

6. Système selon l'une quelconque des revendications 1 à 5, dans lequel le promoteur spécifique d'un tissu de la deuxième cassette est un promoteur spécifique du foie.

7. Système selon la revendication 6, dans lequel le promoteur spécifique du foie est un promoteur d'albumine, un promoteur de globine se liant à la thyroxine, un promoteur de phosphoènolpyruvatecarboxykinase (PEPCK), un promoteur de tyrosine aminotransgérase (TAT), ou un promoteur d'alpha-1-antitrypsine humaine (hAAT).

8. Système selon l'une quelconque des revendications précédentes, pour une utilisation en thérapie.

9. Système selon l'une quelconque des revendications précédentes pour une utilisation dans un procédé de traitement du diabète, de l'obésité, du syndrome métabolique, ou de la lipodystrophie chez un sujet ; dans lequel la première cassette comprend un élément régulateur et un transgène de leptine lié de manière fonctionnelle à un promoteur ; et dans lequel la deuxième cassette comprend un promoteur spécifique du foie lié de manière fonctionnelle à un microARN qui cible l'élément régulateur dans la première cassette d'expression.

10. Système selon l'une quelconque des revendications 1 à 8 pour une utilisation dans un procédé de traitement d'un cancer chez un sujet ; dans lequel la première cassette comprend un élément régulateur et un transgène d'IL-15 lié de manière fonctionnelle à un promoteur ; et dans lequel la deuxième cassette comprend un promoteur spécifique du foie lié de manière fonctionnelle à un microARN qui cible l'élément régulateur dans la première cassette d'expression.

11. Système selon la revendication 9 ou 10, dans lequel l'élément régulateur de la première cassette est une séquence d'élément régulateur post-transcription de marmotte (WPRE).

12. Système selon l'une quelconque des revendications 8 à 11, dans lequel le vecteur AAV est administré par voie intrapéritonéale.

13. Système selon l'une des revendications 9 à 12, dans lequel le promoteur spécifique du foie est un promoteur d'albumine, un promoteur de globine se liant à la thyroxine, un promoteur de phosphoènolpyruvatecarboxykinase (PEPCK), un promoteur de tyrosine aminotransgérase (TAT), ou un promoteur d'alpha-1-antitrypsine humaine (hAAT).
